# EUROPEAN PATENT APPLICATION

(11) **EP 3 261 007 A1**
(43) Date of publication of application: **27.12.2017**
(21) Application number: 16305753.2
(22) Date of filing: 22.06.2016
(51) Int. Cl.: G06F 19/18

(54) **IMPROVED COMPUTER IMPLEMENTED METHOD FOR BREEDING SCHEME TESTING**

(71) Applicant: Vilmorin & Cie, 75001 Paris (FR)
(72) Inventor: DUCROCQ, Sebastien, 63200 RIOM (FR); HESLOT, Nicolas, 63200 RIOM (FR); BOTTRAUD, Jean Christophe, 63000 CLERMONT FERRAND (FR); FLAMENT, Pascal, 63170 AUBIERE (FR); KARAMAN, Zivan, 63400 Chamalières (FR)
(74) Representative: Cabinet Plasseraud

(57) **Abstract**

The invention aims at a computer implemented method for breeding scheme testing, comprising the steps of:
a) Receiving input data through a user interface,
b) Implementing calculation steps related to a simulation of said breeding scheme and based on said input data,
c) Outputting test results data resulting from said simulation,
Wherein:
- Said calculation steps use at least one modular operation selected among a multiplicity of predefined modular operations stored as respective computer routines available in a computer library, and
- Said input data comprise at least an indication of at least one modular operation selected by a user.

## Description

The invention relates to a computer implemented method for breeding scheme testing.

Efficient plant breeding schemes are needed for developing improved plants. A scheme starts with a group of individuals, and a crossing of them is performed to generate new individuals which are further tested for performance *in silico, in vivo* or a combination of both so as to finally select a few individuals identified as superior.

Improved schemes can be proposed using an *a priori* knowledge and tested empirically but the length and cost associated with a real-life scheme evaluation makes it highly impractical. Schemes can be improved by changing the value of a parameter such as the size of the population or by using different operations in a different order. The stochastic nature of the plant breeding process also requires multiple replicates of the same scheme to obtain a robust evaluation of any alternative scheme.

Classical quantitative genetics theory makes a number of simplifying assumptions in order to develop mathematical expressions that describe the mean and variation (genetic and phenotypic) within and among populations, and to predict how these are expected to change under the influence of external forces. Using those simplifying assumptions a number of relatively simple schemes can be evaluated analytically, for example with the so-called "breeder's equation", as disclosed in:
- Endelman JB, Atlin GN, Beyene Y, et al. (2014) "Optimal design of preliminary yield trials with genome-wide markers", Crop Sci 54:48-59 ; doi: 10.2135/cropsci2013.03.0154.
This approach does not extend well to more complex breeding schemes.

The only analytic approach to optimization of complex breeding scheme is perhaps the class of problems termed "gene pyramiding" or "gene stacking" in literature, as disclosed for example in:.
- Servin B, Martin OC, Mézard M, Hospital F (2004) "Toward a theory of marker-assisted gene pyramiding", Genetics 168:513-23 ; doi: 10.1534/genetics.103.023358,
- Canzar S, El-Kebir M (2011) "A mathematical programming approach to marker-assisted gene pyramiding", Algorithms Bioinforma 26-38
- Xu P, Wang L, Beavis WD (2011) "An optimization approach to gene stacking", Eur J Oper Res 214:168-178.

The problem of gene pyramiding is to identify the best way to combine into a single genotype a series of target genes identified in different parents. Assuming that individuals can be selected and mated according to their genotype, the best method corresponds to an optimal succession of crosses over several generations. Few methods have been proposed to address the gene pyramiding problem. A dynamic programming method cannot be very efficient as all pedigrees need to be enumerated and the number of pedigrees is exponential with respect to the number of founding parents. Thus the method is only able to handle relatively small number of initial parents. The population size is to be thus minimized and does not consider other criterions such as the number of generations, etc.

Attempts, based on criterions such as the number of crossings, the number of generations and the population size, have been proposed, turning the optimization problem into a mixed integer programming problem and embedding the structure. An alternative formulation of the optimization problem can be to extend a multi-objective model and to include the number of crossings and the population size as well. Then there 4 optimizations in the objective function can be performed:
(1) maximize the likelihood of successfully obtaining the desired genotype,
(2) minimize the number of generations,
(3) minimize the number of crossings and
(4) minimize the population size.

However, all these optimization problems being of a NP-hard type algorithm, obtaining optimal solutions may require heavy computation and the problem size is generally small. In order to solve large problems, heuristic solutions can be contemplated. However, a heuristic exploitation of genetic structure in marker-assisted gene pyramiding is limited in terms of loci (for example to 10-14 loci), and finally to a very specific problem (pyramiding a few genes).

With the availability of high-speed computers, simulation has been used to test breeding schemes *in-silico* and relax many of the assumptions made by quantitative genetics theory, in particular pleiotropic and epistatic effects and gene by environment interaction. The most widely known publically available software is probably the so-called "Qu-Gene" software, disclosed in:
- Podlich DW, Cooper M (1998) QU-GENE: a simulation platform for quantitative analysis of genetic models. Bioinformatics 14:632-53.

Qu-Gene was developed to simulate most classical breeding strategies for inbred and hybrids crops. Qu-Gene is a simulation software to investigate the characteristics of genetic material undergoing repeated cycles of selection and molecular marking. It is declined in modules for inbred lines (QuLine) and module for hybrid varieties (QuHybrid). It creates a population of genotypes from a genetic model. The model is defined as a specification of the genetic architecture of the trait(s) and how this is influenced by the types of environment that make up the target population of environments. Features available are:
- diploid populations with multiple alleles per gene
- traits influenced by multiple genes, di-genic epistasis and GxE (Genotype by Environment)
- defines and creates populations of any type (pedigree-derived, inbred, hybrid)
- describes and flowcharts breeding programs
- models the results of inbred and hybrid programs (including MAS (Marker Assisted Selection), MARS (Marker Assisted Recurrent Selection, GS (Genomic Selection))
- allows selection based on traits, trait indices, markers, marker scores, marker BLUPs
- runs in multi-processor environments
- integrated with APSIM biophysical model of cropping systems.

However, QU-Gene is not flexible enough to be used for complex scheme optimization. Further, it is not designed to interface with optimization routines and allow only mere comparison of a few schemes. It is declined in different products (one for inbred lines (QuLine), one for hybrid (QuHybrid), one for marker assisted selection (QuMARS) that can't be easily combined. It also does not handle costs and duration of scheme.

More generally, currently available software for breeding optimization are built on breeding simulations for a specific scheme: QU-Gene for example has specific applications related to the breeding context (self or cross-pollinated crop, marker assisted selection etc.).

However, a typical plant breeding scheme can have dozens of parameters making a complete grid search prohibitive. Optimization is needed on the size of the different populations, selection pressure used, crossing design, taking into consideration cost, time, diversity loss, etc. However, optimization is not restricted to those parameters. Structure of the scheme can also be considered such as order of the operations, type of crossing operations, number of cycles of selection, etc. There is a need for efficient search strategies to identify better schemes by optimizing the succession of operations and the parameters of each operation. No solution has been proposed to this problem so far.

The present invention aims to improve the situation.

To that end, the invention aims at a computer implemented method for breeding scheme testing, comprising the steps of:
a) Receiving input data through a user interface,
b) Implementing calculation steps related to a simulation of said breeding scheme and based on said input data,
c) Outputting test results data resulting from said simulation.
More particularly:
- Said calculation steps use at least one modular operation selected among a multiplicity of predefined modular operations stored as respective computer routines available in a computer library, and
- Said input data comprise at least an indication of at least one modular operation selected by a user.

In an embodiment, the method comprises a preliminary step of defining a plurality of blocks stored in said computer library, each block corresponding to a single modular operation or to a cluster of successive modular operations, and each block being callable during step b) so as to perform calculation steps corresponding to modular operations of a called block and in an order defined in that called block.

Furthermore, categories of blocks can be defined during said preliminary step, said plurality of blocks being listed by categories so that one block only among several blocks of a same category can be called during said calculation steps.

In a possible embodiment, the aforesaid input data can comprise a template wherein a list and an order of the categories are defined.

In an embodiment, breeding schemes are successively tested, steps b) and c) being successively repeated with respective different sets of modular operations selected from said computer library. An optimization module is preferably implemented so as to compare results obtained with respective sets of modular operations, according to at least one given criterion, in view to identify at least one set of modular operations generating a superior breeding according to said given criterion.

The aforesaid optimization module selects successively chosen sets of modular operations so as to reach said superior breeding, preferably according to a stochastic approach.

In an embodiment, the optimization module uses results from an implementation of step c) to identify a new set of modular operations to test in a subsequent implementation of steps b) and c).

Furthermore, the optimization module can optimize further a plurality of parameters' values related to distinct modular operations to implement in each step b), in view to generate said superior breeding scheme.

In an embodiment combining the category/bloc construction and the aforesaid optimization module, breeding schemes are successively tested, steps b) and c) being successively repeated, and an optimization module is provided so as to select a different set of chosen blocks at each implementation of step b), and for one implementation of step b) each chosen block belongs to a distinct category.

In that embodiment, the optimization module can compare results obtained with respective sets of blocks at each implementation of steps b) and c), according to at least one given criterion, in view to identify at least one set of blocks generating a superior breeding according to said given criterion.

In that embodiment, blocks of a same category are interchangeable from one implementation of step b) to another, and the optimization module can choose a single block of a same category for one implementation of step b).

The aforesaid given criterion can be chosen among a set of criterions comprising a genetic gain, a genetic gain stability, value of a given agronomic traits for individuals resulting from a tested scheme, total scheme cost, duration, genetic diversity loss, number of crossing-overs, robustness to failure.

Of course, the optimization module can use several criterions of said set of criterions, taken in combination for example optimize a criterion while restricting another within provided limits

The present invention aims also at a computer program product, comprising instructions to perform the method according to the invention, when such instructions are executed by a logical circuit (as presented below referring to figure 5 and exemplary figures 8E and 9D).

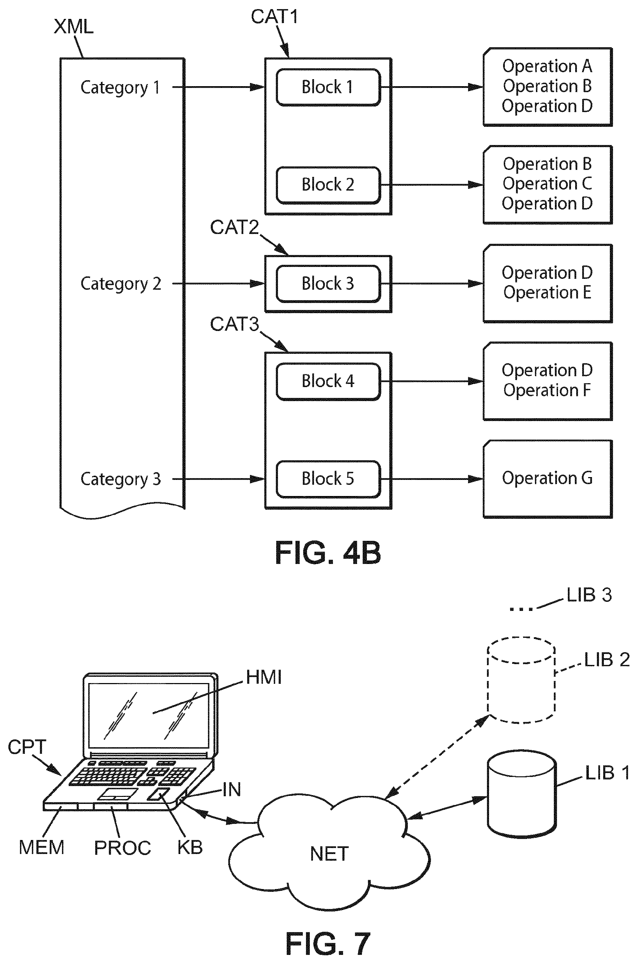

The present invention aims also at a computer device, comprising a logical circuit, connected to a human/machine interface device so as to perform the method according to the invention (as shown on figure 7 commented below).

Therefore, according to a feature of the invention, breeding schemes can be built by combination on unitary operations. By this construction, the breeding scheme structure can be subject to routine optimization. The combination of the simulation software and optimization routine allows the simultaneous identification of the better parameters for each operation and the optimal selection and order of operations.

In addition, the method of the invention is suitable for use with stochastic optimization methods because of the complexity and high dimensionality of the search space. Because of the size of the search space, it cannot be fully explored by grid search. Computing performance is thus also critical, and the invention provides a solution hereby by using a distributed computing solution.

The present invention is illustrated by way of example, and not by way of limitation, in the figures of the accompanying drawings, in which like reference numerals refer to similar elements and in which:

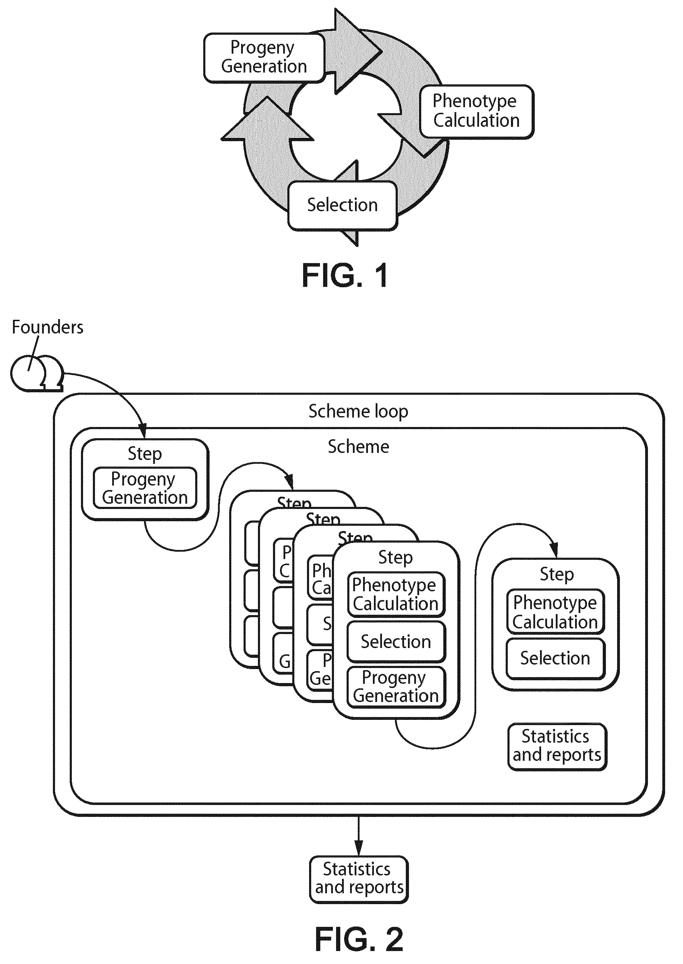

- Figure 1 shows a usual basic breeding loop,
- Figure 2 is a plant breeding plan simulation overview,

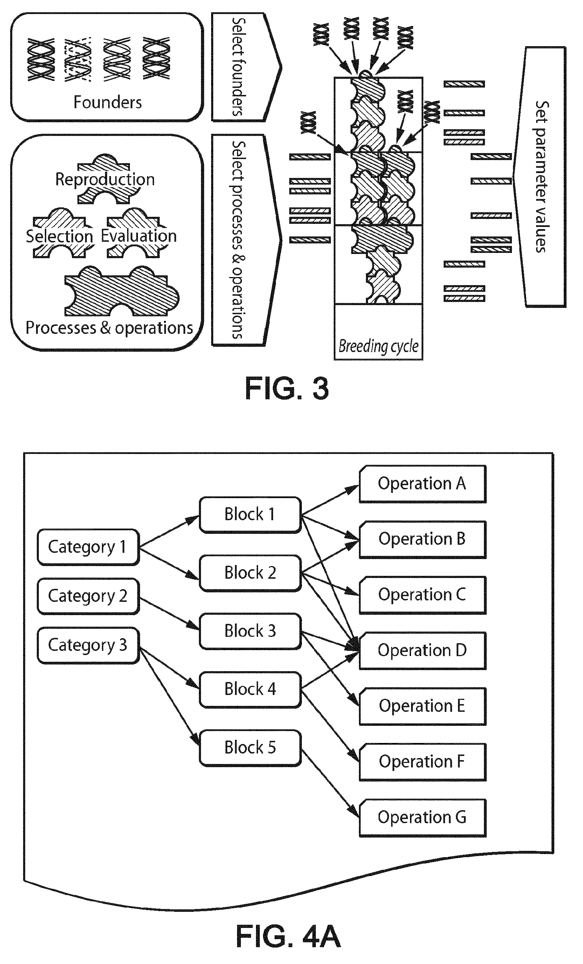

- Figure 3 shows schematically a breeding scheme optimization,
- Figure 4A shows an example of the content of each block, in each contemplated category,
- Figure 4B shows, in that example, built links between blocks and respective modular operations (A, B, C, etc.), and categories and corresponding possible blocks,

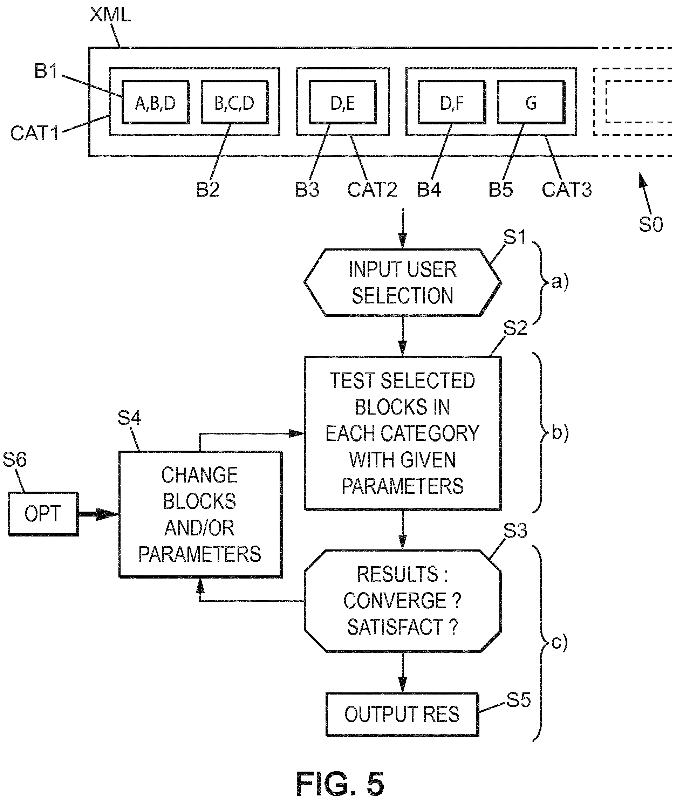

- Figure 5 shows an example of a general method according to the invention,

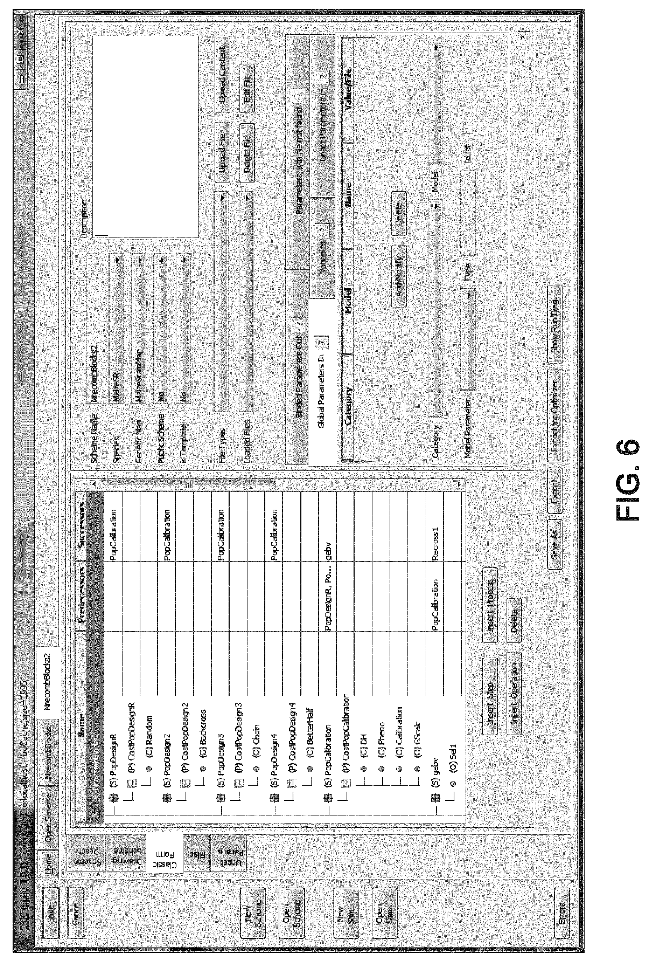

- Figure 6 shows a possible human/machine interface screenshot as an example so as to perform step S1 of figure 5,
- Figure 7 shows an example of a system including at least one device for implementing the method of figure 5,

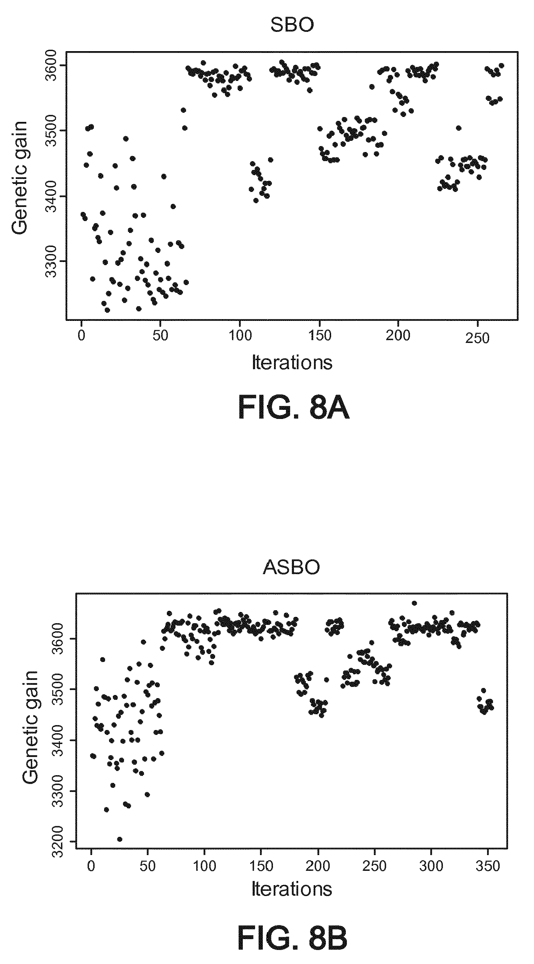

- Figure 8A shows a first example of simulation and more particularly a resulting genetic gain (number of wanted alleles) versus a number of iterations of the optimization process, using an SBO optimization algorithm,
- Figure 8B shows the same first example of simulation and more particularly the resulting genetic gain (number of wanted alleles) versus a number of iterations of the optimization process when using an ASBO optimization algorithm,

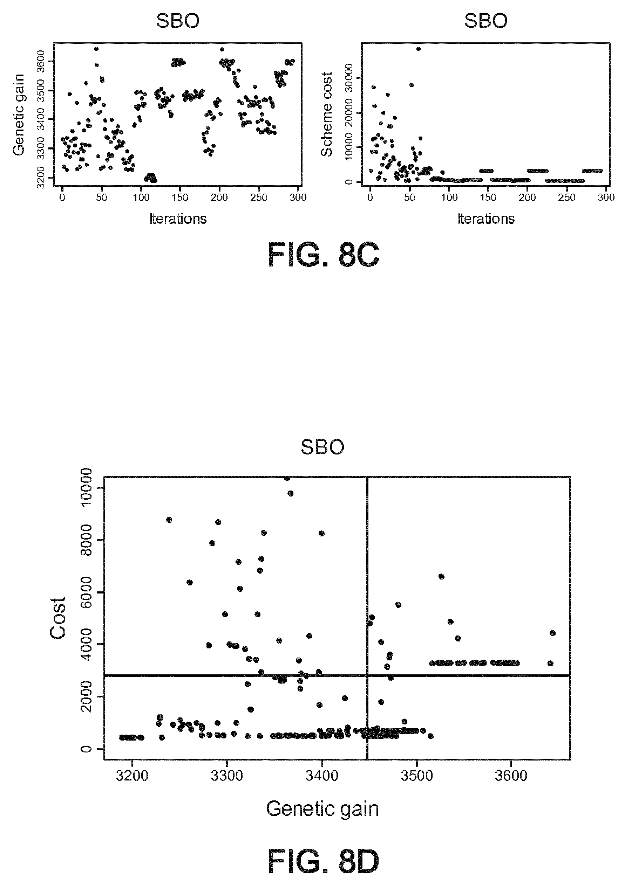

- Figure 8C shows respectively the genetic gain (left part) and the total cost of each scheme (right part) versus the number of the needed iterations,
- Figure 8D shows a plot resulting from the cost versus the genetic gain, showing thus previously unrevealed but very interesting possible breeding schemes in the lower right corner of figure 8D (revealed thanks to the optimization according to the method of the invention), dark lines indicating baseline cost and genetic gain for the corresponding scheme used by practitioners,

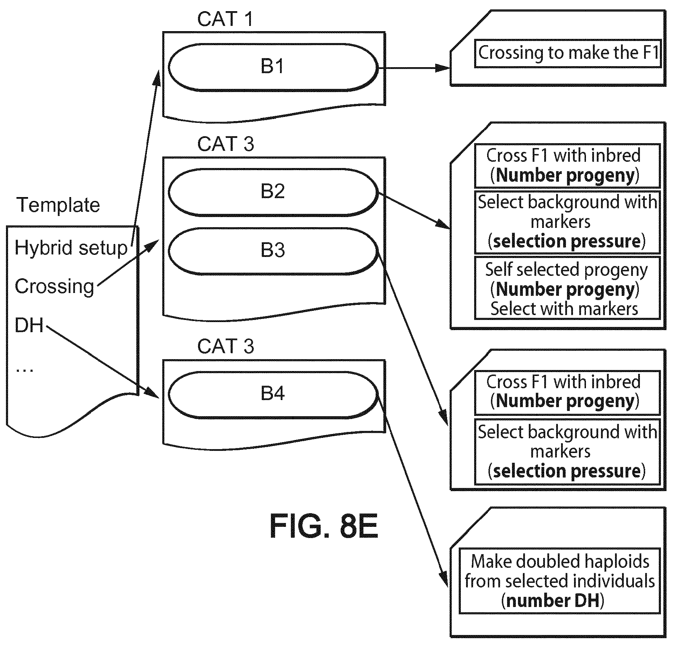

- Figure 8E shows categories and blocks architecture used for performing the simulations in the first example on transfer of one or several chromosomes from one background to another (bold characters aim at the numerical parameters subject to optimization in addition to block selection),

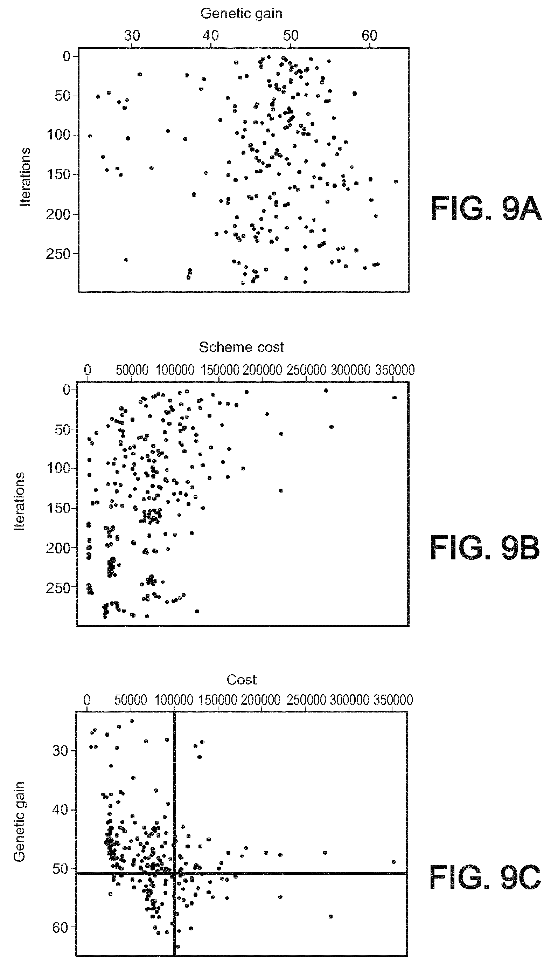

- Figure 9A shows a second example of optimization on a recurrent selection scheme with genomic selection and more particularly a resulting genetic gain versus a number of iterations of the optimization process, using here a genetic algorithm optimization algorithm,
- Figure 9B shows the total cost of each scheme versus the number of the needed iterations,
- Figure 9C shows a plot resulting from the cost versus the genetic gain, showing thus interesting possible breeding schemes in the lower left corner (revealed thanks to the optimization according to the method of the invention), dark lines indicating baseline cost and genetic gain for the corresponding scheme used by practitioners,

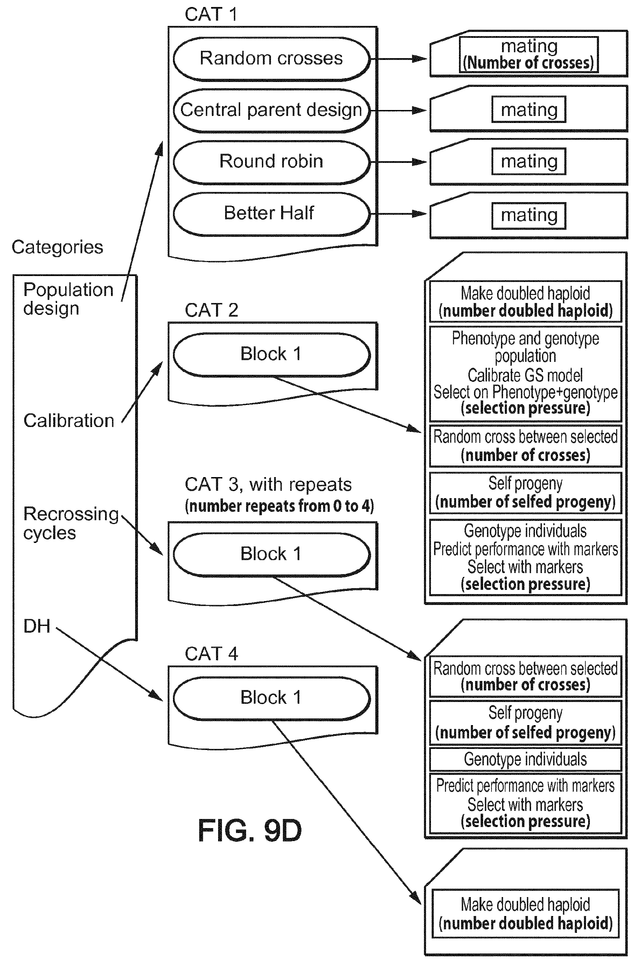

- Figure 9D shows categories and blocks architecture used for performing the simulations in that second example (bold characters aim at the numerical parameters subject to optimization in addition to block selection).

To optimize plant breeding scheme efficiently, a simulation tool has been developed with a partition of the breeding schemes in blocks (called "library" hereafter). Some of these libraries include:
- modelling of cross to perfectly mimic real life schemes,
- modelling of the stochastic nature of field trials: stochastic sampling of environments, loss of trials/plots.
Specific models to use in the simulator tools can be used for selection decisions, such as a marker-assisted selection software. The specific organization of the simulation tool, as a library of blocks, is flexible enough to allow integration by users of their own selection scheme but also other additional modules such as analysis tools used by breeders (performance evaluation, selection decision, etc.).

This tool allows modeling scheme operations and optimization of parameters as number of individuals, number of generations, replicate, time, cost, etc. but also modeling of the stochastic nature of plant breeding field trials, sampling of environments in the target population of environments. The tool can model further realistic cases, and can handle very high population sizes and marker densities at a reasonable computing cost and time. The tool can model complex biological phenomenon related to trait architecture (epistasis, genotype by environment interactions).

Such simulation organizations allow further integration of stochastic optimization strategies to solve generic breeding scheme optimization problems (structure of the scheme) and allow simultaneous optimization of both parameters and structure of schemes. Computation can be distributed at the level of the repetition of a scheme or by splitting the operations of the scheme to increase efficiency and handle very large populations. Optimization can be done over multiple criteria or combination of criteria including genetic gain, cost, duration, loss of genetic diversity, sensitivity of the scheme to input individuals. Surrogate based optimization (SBO) and genetic algorithm) using libraries such as PYGA (for "Python Genetic Algorithm") are examples of stochastic optimization routine that can be interfaced to the tool. The routine optimization can be run independently to compare result or successively to get the better chance to have an optimal result.

To limit infrastructure cost and to ensure computational efficiency, it can be used a sparse representation of genetic information as segments of the original founders combined with the recording of crossing over to keep track of the contribution of a segment to a trait estimate, and to factor out that computation across generations. A corresponding implementation is disclosed in documents US-9,041,566 and US-2014/0136161.

The computation can be distributed on generic servers with the possibility to easily increase the infrastructure size as needed.

Simulation tool and routine optimization are included in a computer program with user interface to catalog of operations, pre-built schemes, easily run simulations and share results. Intermediate results of a scheme can be made available to the user through the interface.

The design and development of a plant breeding scheme simulator is part of the overall solution to optimize breeding strategies and of the overall breeding decision process. Advantageously, the simulation tool provides further a way to simulate the different processes involved in a plant breeding scheme. Roughly speaking, a breeding program or scheme consists of a regime of possible progeny generation steps, alternating different strategies (crossing/selfing/DH (for "doubled haploids" plant)), followed by selections based on a set of parameter values on phenotype traits of individuals, or sets of individuals, or on their marker genotypes.

A basic breeding loop, as shown on figure 1, consists of selection, progeny generation, and progeny value evaluation, which is equivalent to the real life process of making the seeds, planting the seeds, growing the plants and measuring trait values.

A plant breeding schema is made of steps, each step corresponding to one iteration of the basic breeding loop described above. Each step includes potentially three processes:
- A progeny generation process (a new plant population being generated from a parent population),
- An evaluation process which evaluates the phenotype of the progeny generated in the previous generation process (in this step or in the previous one).
- A selection process (to select for instance a subset of progenies from the current population based on the result of the evaluation process).

Additional processes may be added to export data, to compute statistics and to create reports. Furthermore, the order of the processes within a cycle may vary. Indeed, in most schemes the first step will start with a progeny generation, using a set of founders given as input to the scheme, and the last step will end either with progeny value computation or a selection.

Finally, figure 2 gives an overview of a selection scheme.

The user is able to define for each generation (or step) the module operation (crossing operation, selection, etc.) and its parameters. A species and founders (individuals given in scheme entry, real individuals or simulated individuals) are defined by the user and provided as input to the simulation tool along with a genetic map and a genetic architecture. A genetic architecture is defined for a trait by the position of causal loci, their effects, interactions between causal loci if any (epistasis), heritability, interactions between causal loci and the environments if any.

The simulation must satisfy biological constraints, such as the maximum number of progenies that can be derived at each generation as specified by the species parameters, the time of year when a specific operation can occur, the duration of an operation, etc.

The description of a scheme and its use in a simulation requires several operations:
- First the user needs to describe a scheme,
- The user then must describe the use of a scheme in a simulation,
- Finally, the user can run a simulation and browse the results.

The optimization of breeding schemes can be separated into three sub-problems as presented in Figure 3:
- The selection of individuals to start the scheme,
- The construction of an optimal scheme, and
- The optimization of the scheme parameters.

One of the problems addressed by the invention is related to the construction of an optimal scheme and the optimization of the scheme parameters.

The input of the simulator can include:
- Configuration input describing general governing optimization parameters,
- Parameter input describing optimized variables,
- Scheme input describing how a user has to provide a scheme for optimization (partially referring to parameter input), containing the library of blocks to be used and how they can be combined,
- Objective input defining targets (partially referring to parameter input).

The scheme is described using scheme template and blocks, as detailed below.

A so-called "block" is a list of modular operations or groups of modular operations, of one or more modular operation. To construct a block for a scheme, the user specifies most of the information that exist in the scheme. Some information used in scheme may be omitted, since it can be automatically computed or filled by the block mechanism on the stage of constructing the executable scheme.

Furthermore, blocks are classified within categories. A category represents a biological function. Each category may contain a list of blocks doing (biologically) the same function in different ways. Blocks defined in the same category are interchangeable and can be switched by an optimization module. The user provides (in the global parameters file) a path of a file containing all blocks.

In addition to the switchable blocks, a category allows to define the inputs and outputs that must be defined in each block. The input (and output) must be defined in the block by adding an attribute "category Parameter" in the corresponding parameter. The value of this attribute is the name of the input (or output). Defining inputs and outputs of blocks allows the simulator and its optimization module to create automatically links between the chosen blocks.

Figure 4A shows an example of the content of each block, in each category, and figure 4B shows a corresponding category/block/operation architecture.

In that example, it should be understood that, if category 2 is used, then just one single block (block 3) can be used, without any other possible alternative. On the opposite, if category 1 is used, either block 1 or block 2 can be used. In the same way, if category 3 is to be used, one of the blocks 4 or 5 can be selected. For example, if an optimization process is performed to test successively several breeding schemes, one exemplary possible way is to use then block 3 with the following successive combinations:
- block 1, block3 and block 4,
- block 1, block3 and block 5,
- block 2, block3 and block 4,
- block 2, block3 and block 5.
Therefore, modular operations A, B, C, D, etc. are gathered in predefined blocks according to practical breeding rules, and those predefined blocks are listed per category, according also to practical breeding rules. This is a first step S0 (a preliminary step) of a general process shown on figure 5.

That provision makes it possible to limit the number of combinations of modular operations to test, and thereby to reduce drastically needed computer resources.

Then, during the following of the process as shown on figure 5, a first current step S1 is related to the reception by a computer machine of the input of a user through a human interface device (a tactile screen, a keyboard or a click mouse and a screen, etc.), as shown further in the example of figure 6.

Referring to figure 6, a user can select possible blocks (PopDesignR, and PopDesign3 for example of the category "crossing" grouping the first four blocks) in each category of the left part of figure 6. The user can input further chosen parameters in the right side of the given example of figure 6 (for example a particular type of plant, or possibly also a wished phenotype trait such as plant height, number of seeds per ear, etc.).

To provide a complete scheme, the user specifies a template which corresponds, in an exemplary embodiment, to an XML file (e.g. "sampleTemplate.xml") giving the list of categories corresponding to one step in the simulation scheme, their order and possibly repeats along with additional information that is used to generate a valid simulation scheme.

For each operations included in a block definition, duration, cost and time constraint can be defined and associated with a process. For example, for the matter of cost, an evaluated cost can be defined to be proportional to the number of individuals for a specific operation.

Each process can be included then in a block definition. In that sample, a process XML node is created in the scheme. The process is associated to an operation ("MakeDH" for example) and set proportional to its "parentList" parameter. The given "name" of the process corresponds to a name in the presented cost and duration catalogue (given in global or specific parameter input file).

Furthermore, an input of an aim to reach defines an objective to optimize. As objective input the user provides observables (components) and objectives (aims) that are constructed from components. Objective can be built as a combination of any parameter subject to optimization and values from the breeding simulator reports. They can include genetic gain on one or several trait, duration of the scheme, cost of the scheme, diversity loss, etc. It is possible to specify a multi-objective problem (e.g. genetic gain and cost) or to add constraints (e.g. maximize genetic gain with a budget below a certain threshold)

That step S1 corresponds to the previously labelled "step a)".

In step S2 (corresponding to step b)), the test is performed with selected blocks and parameters (or predefined blocks and parameters tested successively if they have not been inputted by the user). Actually, several tests are performed with parameters or blocks which were not specified by the user. More particularly, the optimization module S6 selects successively more adapted parameters and/or more adapted blocks in step S4 so as to reach, according to a stochastic approach, a converging solution in step S3. That converging solution can be a scheme improving the likelihood of obtaining a type of plant having a particular phenotype trait specified by the user (defined objectives), or as for another example a robust plant type according to specified parents and a particular environment for example (defined components).

The tests are performed according to parametric optimizations in an example of embodiment. The optimization module can further:
- change the number of modular operations to perform at each implementation of step S2 (with minor structure variation, sequential of similar groups of operations being previously defined in the predetermined blocks), and/or
- substitute operations (they may be not explicitly parameterized, but they need only to be put in one given block file), and/or
- add or remove blocks.
The corresponding optimization problems can be handled both by SBO and GA (for "Surrogate Based Optimization" and "Genetic Algorithm").

In step S5, the final results (or even intermediary results obtained after each implementation of step S2) can be outputted through a human/machine interface. Here, steps S3 and S5 correspond to "step c)" as previously labelled.

Referring now to figure 7, a computer system which can be used for implementing the method can include:
- one or several servers LIB1, LIB2, LIB3, ..., each one having at least one memory for storing a library comprising a multiplicity of modular operations organized per predefined blocks and categories, as described above (the libraries of the different servers being compatible from one to another, and defining especially the organization per blocks and categories in a same way),
- a computer device CPT connectable to the servers through a telecommunication network NET, and comprising a human/machine interface (HMI, comprising a screen, a keyboard KB, etc.) so as to input user's selections and connected to a logical circuit comprising a processor PROC, a working memory MEM, and a communication interface INT through the network NET.

In an embodiment, the computer device CPT can be connected to one or several online libraries LIB 1, LIB2, ... and a user can select categories and possibly blocks in these categories through the interface HMI, so as to download from the servers the useful modular operations defined in these blocs. These operations can be calculated in the logical circuit of the computer device (e.g. the processor PROC executing then a computer program according to the invention and the memory MEM storing the instructions code of that program). However, in a possible alternative embodiment, the calculations can be performed in one or several servers LIB1, LIB2, etc. (preferably in parallel so as to minimize the calculation time) and the computer device CPT simply displays downloaded results of the servers calculations. Then, the logical circuit for running the computer program of the invention can be localized equivalently either in a local computer device CPT (such as a laptop, a tablet, or any other terminal device), or in remote server LIB1, and/or LIB2, etc.

Examples of implementation of the method of the invention are given in details below.

A first example concerns the transfer of one or several chromosome from one background to another.

In this use case, the objective is to identify the most efficient breeding scheme starting with two populations (e.g. two maize heterotic groups) called A and B hereafter and recover an inbred line with mostly "A" alleles and zero to n "B" chromosomes from an F1 hybrid between an A and B inbred line. The scheme starts with the F1 of an A and B inbred line followed by a cross with the same A inbred line, another inbred line or another hybrid derived from A and B inbred line. Alternatively, the scheme can start from preexisting F1, and possibly parental alleles are inferred within a combination of statistical and genotyping methods. Progenies are selfed with one or two generations and sorted using markers selected to distinguish A from B background.

Parameters to optimize in that scheme are:
- the initial population size of selfed progeny,
- the number of plants selected from this population (selection pressure),
- the number of cycles of selection,
- if there is another cycle of selection, the number of selfed progeny to generate per selected individual and the selection pressure in that cycle,
- finally, the number of doubled haploid lines to derive per selected line at the end of the process.
Objectives are:
- maximizing recovery of the A background on the chosen chromosomes while minimizing scheme duration and total cost,
- robustness of the scheme can also be included in the objective by combining mean and standard error of the measure of the background recovery.

For that scheme, "real life values" used by practitioners and based on experience are also available and were used for comparison purpose.

Figures 8A and 8B show the behavior of the genetic gain related to the number of iteration of algorithm using SBO algorithm and respectively another optimization algorithm called "adaptive SBO". Adaptive SBO integrates a measure of uncertainty of the objective function to better allocate computing resources between testing more schemes or evaluating more thoroughly fewer schemes. The X axis is related to the number of iterations of the algorithm and the Y axis is related to the objective function (here recovery of A alleles on the wanted chromosome / number of alleles).

Therefore, the algorithm converges on optima and the identified best scheme is nevertheless 6% better in terms of recovery of A alleles than the baseline scheme. Similar results can be observed with a different optimization method "ASBO" (for adaptive SBO) as shown on figure 8B.

To be closer to practice, the optimization can be done on two objectives simultaneously:
- maximize recovery of wanted alleles and
- minimize cost.
The average cost of real life scheme is 2795 euros.
Figure 8C shows respectively the genetic gain (left part) and the total costs of schemes (right part) versus the number of the needed iterations.

If both objectives are plotted as shown on figure 8D (cost versus genetic gain), the dots in the lower right corner corresponds to schemes that are better than current scheme in terms of allele recovery and cost. To evaluate the schemes obtained with the method, they were compared to schemes obtained with small variations around "real life values". On average for those schemes, the average cost obtained is 3448 euros. To ensure the identification of a robust scheme, 10 "bundles" were used simultaneously in the optimization. A bundle is a set of input files and parameters needed to evaluate a scheme. A bundle would contain for example a genetic map, a genetic architecture, genotype data of founders. For the optimization, using several bundles help in identifying reliable schemes. The straight lines are then the mean of the schemes "values" which are close to a current not optimized scheme (a cost of 2795 euros, and a genetic gain value of 3448). Those straight lines are the average of a baseline scheme. However, many better schemes appear thanks to the simulation method in the lower and right corner of figure 8D.

With the best scheme in that example, the cost efficiency is improved 2.67 folds over the current scheme. A table providing default values for the scheme parameters and optimized schemes can be outputted and displayed (or printed) for a user.

Figure 8E shows the template (choices of categories) and blocks which were used in that first example. The operation parameters which were subjects to optimization in addition to the block choice are shown in bold characters.

A second example of embodiment for the use of the method of the invention is described below relatively to optimization of recurrent selection with genomic selection. In that scheme, a population of doubled haploid lines is generated from a population of few founders, phenotyped and genotyped. Best individuals are selected based on phenotype plus genotypes and re-crossed followed by several rounds of "marker only" selection. There is up to 15 parameters to simultaneously optimize, such as:
- Type of crossing schemes used to generate the initial population from a few founders,
- Number of DH to generate initially,
- Initial selection pressure (genotype + phenotype for example),
- Number of crosses to make in the first round of re-crossing,
- Number of selfed progeny to generate from the first round of re-crossing,
- Selection pressure in further rounds of marker only selection,
- Number of cycles of marker only selection,
- Number of crosses to make in further rounds of marker only selection,
- Number of progeny per cross in further rounds of marker only selection, and
- Number of DH to generate per selected entries at the end of the scheme.

The objective is to maximize genetic gain with a constraint on time and cost or maximizing genetic gain per unit cost while identifying robust schemes.

Figures 9A and 9B result from an optimized simulation using the genetic algorithm with respective focus on maximizing genetic gain and minimizing the scheme cost.

If both objectives are plotted as shown on figure 9C, the straight lines being the average of the baseline scheme, the dots in the lower left corner corresponds to scheme that are better than currently used scheme in terms of genetic gain and cost.

With the baseline, one unit of genetic gain requires 1795 euros while only 172 euros are required with the optimal one, corresponding to a 10 fold improvement in breeding efficiency.

Figure 9D shows the used template and blocks for that second example of embodiment.

The invention provides thus a computer implemented breeding simulation method, making possible to define for a given breeding objective the best breeding strategy to use. It involves an interfaced breeding simulator and preferably an optimization routine. It allows further identification of the most efficient breeding scheme with optionally an optimization of the scheme structure itself. Advantageously, optimized routines are run several successive times, each optimized routine being different from a previous one. Each run time will provide a separate optimized scheme that can be advantageously compared to check convergence of the optimization.

The method according to another possible definition could be summarized as follows:
- Input all available breeding schemes in a library (or connected libraries) of a modular breeding simulator,
- Input all agronomic and genetic data in the breeding simulator,
- Input breeding objective or multi-objectives and constraints (by the user),
- Run the breeding simulator,
- Run one or several times optimized routines on the breeding simulator,
- Collect the optimized breeding scheme to reach the breeding objectives under the defined constraints.

## Claims

1. A computer implemented method for breeding scheme testing, comprising the steps of:
a) Receiving input data through a user interface,
b) Implementing calculation steps related to a simulation of said breeding scheme and based on said input data,
c) Outputting test results data resulting from said simulation,
Wherein:
- Said calculation steps use at least one modular operation selected among a multiplicity of predefined modular operations stored as respective computer routines available in a computer library, and
- Said input data comprise at least an indication of at least one modular operation selected by a user.

2. The method of claim 1, comprising a preliminary step of defining a plurality of blocks stored in said computer library, each block corresponding to a single modular operation or to a cluster of successive modular operations, and each block being callable during step b) so as to perform calculation steps corresponding to modular operations of a called block and in an order defined in that called block.

3. The method of claim 2, wherein categories of blocks are defined during said preliminary step, said plurality of blocks being listed by categories so that one block only among several blocks of a same category can be called during said calculation steps.

4. The method of claim 3, wherein said input data comprise a template wherein a list and an order of the categories are defined.

5. The method according to anyone of the precedent claims, wherein breeding schemes are successively tested, steps b) and c) being successively repeated with respective different sets of modular operations selected from said computer library, and wherein an optimization module is implemented so as to compare results obtained with respective sets of modular operations, according to at least one given criterion, in view to identify at least one set of modular operations generating a superior breeding according to said given criterion.

6. The method of claim 5, wherein said optimization module selects successively chosen sets of modular operations so as to reach said superior breeding according to a stochastic approach.

7. The method of claim 6, wherein the optimization module uses results from an implementation of step c) to identify a new set of modular operations to test in a subsequent implementation of steps b) and c).

8. The method according to anyone of claims 5 to 7, wherein said optimization module optimizes further a plurality of parameters' values related to distinct modular operations to implement in each step b), in view to generate said superior breeding scheme.

9. The method of claim 3 or 4 , wherein breeding schemes are successively tested, steps b) and c) being successively repeated, and wherein an optimization module is provided so as to select a different set of chosen blocks at each implementation of step b), and for one implementation of step b) each chosen block belongs to a distinct category.

10. The method of claim 9, wherein said optimization module compares results obtained with respective sets of blocks at each implementation of steps b) and c), according to at least one given criterion, in view to identify at least one set of blocks generating a superior breeding according to said given criterion.

11. The method of claim 10, wherein blocks of a same category are interchangeable from one implementation of step b) to another, and wherein said optimization module chooses a single block of a same category for one implementation of step b).

12. The method according to any of claims 5 to 8, and 10 and 11, wherein said at least one given criterion is chosen among a set of criterions comprising a genetic gain, a genetic gain stability, value of a given agronomic traits for individuals resulting from a tested scheme, total scheme cost, duration, genetic diversity loss, number of crossing-overs, robustness to failure.

13. The method according to claim 12, wherein said optimization module uses several criterions of said set of criterions, taken in combination.

14. A computer program product, comprising instructions to perform the method according to any of the precedent claims, when such instructions are executed by a logical circuit.

15. A computer device, comprising a logical circuit, connected to a human/machine interface device so as to perform the method according to any of the claims 1 to 13.
